# EUROPEAN PATENT APPLICATION

(11) **EP 4 063 493 A1**
(43) Date of publication of application: **28.09.2022**
(21) Application number: 20891338.4
(22) Date of filing: 19.11.2020
(51) Int. Cl.: C12N 5/0784, C12N 5/10, C12Q 1/02, G01N 33/15, G01N 33/50, G01N 33/53, G01N 33/68

(54) **METHOD FOR EVALUATING SAFETY OF SUBSTANCE IN VITRO USING HUMAN IMMORTALIZED MYELOID CELLS**

(30) Priority: 19.11.2019 JP 2019208711
(71) Applicant: Mican Technologies Inc., Kyoto-shi, Kyoto 615-8245 (JP); Nissui Pharmaceutical Co., Ltd., Tokyo 110-8736 (JP)
(72) Inventor: MIYAZAKI, Kazuo, Kyoto-shi, Kyoto 615-8245 (JP); SHIMIZU, Jun, Kyoto-shi, Kyoto 615-8245 (JP); TANAKA, Yoshitaka, Yuki-shi, Ibaraki 307-0036 (JP)
(74) Representative: J A Kemp LLP
(86) International application number: PCT/JP2020/043272
(87) International publication number: WO 2021/100828

(57) **Abstract**

[Problem]

To find a method having higher stability, reproducibility, economic efficiency, and operation easiness in an evaluation method of safety of a substance in vitro, by using human immortalized myeloid cells.

[Solving Means]

A method for evaluating the skin sensitizing property and/or the pyrogenic property of a test substance, a method for detecting a skin sensitizer and/or a pyrogen in a sample, and a method for evaluating the action of a sample on a function of immune cells, each using human immortalized myeloid cells and including measuring the production amount of IL-6 and/or IL-8 in a culture medium of human immortalized myeloid cells.

## Description

### [CROSS REFERENCES TO RELATED APPLICATION]

This international application claims priority to Japanese Patent Application No. 2019-208711, filed on November 19, 2019, which is incorporated herein by reference in its entirety.

### [Technical Field]

The present invention relates to a method for evaluating skin sensitizing property and/or pyrogenic property of a substance in vitro, a method for detecting a skin sensitizer and/or a pyrogen in a sample, and a method for evaluating an action of a sample on the function of immune cells, each using human immortalized myeloid cells.

### [Background Art]

When providing a product or substance to be eaten or applied by humans and the like, it is necessary to recognize in advance whether the substance contained in the product is safe and does not cause an allergic reaction and the like.

As a test method therefor, (for example, as a method for evaluating whether a substance induces allergy, etc.), a method including administering or applying the substance to an experimental animal such as mouse or guinea pig, and observing an inflammatory reaction and the like in the skin of the experimental animal has been conducted. In addition, as a test for confirming whether foods, cosmetics, and the like are contaminated with viruses, bacteria, and the like, a pyrogenic property test using rabbits is available. However, from the viewpoint of protection of animals and the like, there is a remarkable tendency toward reduction of the pain to the animals to be used and reduction of the number of animals to be used, and shift to an alternative method is demanded.

As methods for evaluating skin sensitizing property, Guinea Pig Maximization Test (GPMT), Adjuvant and Patch Test (A&P), Buehler Test (BT), Local Lymph Node Assay (LLNA), and the like that use experimental animals have been established so far.

In GPMT, A&P, BT tests, for example, guinea pig is used as an experimental animal and, after sensitizing the animal by several times of subcutaneous administration or application in occlusion of a test solution of a substance dissolved therein, the test solution is applied again in occlusion, the test solution is removed, the skin reaction is observed, and the presence or absence of skin sensitizing property is evaluated by scoring erythema and edema.

In the LLNA test, mouse is used as an experiment animal and, after applying a test solution several times to the auricle of the mouse, a radiolabeled maker substance (e.g., 3H-thymidine) is intravenously administered, the uptake of the maker substance in the lymph node of the auricle is measured, and the presence or absence of skin sensitizing property is evaluated with the activation of lymphocyte proliferation as an index.

As mentioned above, a test using rabbits has been established as a method for evaluating pyrogenic property tests. After measuring the rectal temperature using a temperature sensor in advance, a test solution is administered to the auricle vein and the like of the rabbit, the body temperature rise is measured several hours later, the degree of rise is determined based on the criteria, and whether a pyrogenic substance is contained is evaluated.

In recent years, 3R for protection of animals (replacement of experimental animals with other evaluation methods (Replacement), reduction of the number of animals used (Reduction), and reduction of pain to animals) has been recommended mainly in Europe. Furthermore, since 2009, animal experiments for cosmetics and materials therefor have been completely banned in Europe, and a law has been enacted that prohibits sales of products involving animal experiments. Therefore, in recent years, evaluation methods that replace animal experiments have been developed and reported.

For example, skin sensitization is a reaction that occurs when a causative substance comes into contact with and penetrates the skin. The mechanism thereof is that 1) the substance contacts the skin, 2) the substance is transdermally absorbed, 3) it binds to proteins and is phagocytosed by dendritic cells, 4) the dendritic cells after phagocytosing are activated and migrate to regional lymph nodes, and 5) T cells receive antigen presentation from dendritic cells in the regional lymph nodes, followed by activation and proliferation of the T cells. When these steps occur, immune induction occurs, and when the same substance contacts again, inflammatory cytokines are released from the proliferated T cells, and an allergic inflammation reaction (erythema or edema) is induced.

Based on this mechanism, alternative tests for in vitro evaluation have been developed. For example, Direct Peptide Reactivity Assay (DPRA) is a method for measuring the ease of binding between a protein and a substance by liquid chromatography. In addition, human Cell Line Activation Test (h-CLAT) is a method for measuring the presence or absence of the expression of an activation marker in the dendritic cell activation step, with a flow cytometer by using THP-1 cells. These are animal-free methods.

h-CLAT is an alternative method of a skin sensitization test listed in the guidelines of the Organization for Economic Co-operation and Development (OECD) in 2016. It was found by Shiseido Co., Ltd. and others that the amount of cell surface protein of CD86 or CD54, and MHC Class II, of Langerhans cells which are antigen-presenting cells in the skin, increases due to skin sensitizers, and alternative methods were developed utilizing this phenomenon. However, since Langerhans cells exist only in the epidermis and stable supply of Langerhans cell-like dendritic cells is difficult, an alternative method h-CLAT using human mononuclear cells (THP-1) was established.

As an alternative method replacing experimental animals, h-CLAT is highly useful because it does not use animals (mice) for evaluation and the test period is short, as compared with, for example, LLNA evaluation. However, as an in vitro evaluation method, the operation is complicated and many restrictions are imposed as compared with other constructed methods such as DPRA. For example, preparation of THP-1 cells and grasp of addition concentration by a preliminary test are required in advance, and a flow cytometer is used for measuring surface markers. Furthermore, problems exist in that continuous measurement of positive control and medium control is necessary and that reproducibility is not high (about 80%) (Non Patent Literature 1).

In addition, THP-1 cell does not have sufficient oxidoreductase cytochrome P450, which is activated by differentiation from monocytes, and skin sensitizing property caused by oxidation or reduction of a substance cannot be detected (Non Patent Literature 2).

Therefore, a method that can evaluate the skin sensitizing property of a substance in vitro easily and stably as compared with h-CLAT has been demanded.

Alternative methods replacing experimental animals have also been developed in pyrogenic property tests. A method that can determine contamination relating to Gram-negative bacteria by using the blood of horseshoe crab, which is not a mammal, and a measurement method utilizing Mono-Mac-6 cells and human peripheral blood derived from human acute monocyte leukemia, and utilizing production of cytokine such as IL-6 and the like by stimulation with a sample possibly contaminated with bacteria and the like have been developed.

However, only gram-negative bacteria can be detected using horseshoe crab blood. In addition, Mono-Mac-6 shows lower sensitivity than human blood. Even though the human condition can be evaluated in vitro using human peripheral blood, stable supply is difficult because the blood obtained by blood donation, etc. is utilized, and individual difference may occur.

Therefore, a method for detecting a skin sensitizer and/or a pyrogen by using tissues or cells that are close to human dendritic cells and supplied stably in large amounts has been demanded.

While the function of immune cells is exerted by the released cytokines, there has been no system that can test the direct action of drugs, foods, etc. on immune cells.

For example, there is a method of evaluating an action of a food material on the function of immune cells by using human peripheral blood. However, stable supply is difficult because the blood obtained by blood donation, etc. is utilized, and individual difference may occur.

Therefore, a method that can evaluate the functionality in vitro and achieves easy and stable supply has been demanded.

### [Citation List]

### [Non Patent Literature]

[NPL 1]
   HIFU KANSASEI SHIKEN HYOKA HOKOKUSHO (evaluation report on a test for skin sensitization) JaCVAM editorial committee: skin sensitization test
[NPL 2]
   Takao Ashikaga et al., ATLA 38, 275-284, 2010

### [Summary of Invention]

That is, the present invention provides a method for evaluating the skin sensitizing property and/or the pyrogenic property of a substance in vitro, a method for detecting a skin sensitizer and/or a pyrogen in a sample, and a method for evaluating the action of a sample on a function of immune cells, and aims to find a method having higher stability, reproducibility, economic efficiency, and operation easiness.

### [Solution to Problem]

In pursuit of a method for providing a cell that replaces animal experiments and is suitable for the evaluation of skin sensitizing property and/or pyrogenic property in vitro, the detection of skin sensitizer and/or pyrogen, and the evaluation of an action on the function of immune cells, the present inventors have investigated using monocytes and dendritic cells prepared by various methods. As a result, they have found that the evaluation of skin sensitizing property and/or pyrogenic property, the detection of skin sensitizer and/or pyrogen, and the evaluation of an action on the function of immune cells can be performed using proliferative human monocytes obtained by introducing gene into CD14-positive cells derived from human umbilical cord blood, ES cell, iPS cell, bone marrow cell, or peripheral blood, and cells with phagocytic ability (e.g., dendritic cells) obtained by inducing differentiation of the monocytes, and completed the present invention. In particular, by using these cells, 1) the cells can be provided stably, 2) the cells in a uniform state (passage number, differentiation state) can be provided, and 3) reactions closer to those in the living body can be detected. In the evaluation of skin sensitizing property and/or pyrogenic property, as well as evaluation of an action on the function of immune cells, the present inventors have focused on the production of cytokines (IL-6, IL-8, etc.) caused by the addition of substances and provided novel methods for skin sensitizing property test and/or pyrogenic property test, as well as evaluation of an action on the function of immune cells.

Furthermore, the present invention solves these problems by immortalizing and using CD14-positive cells derived from human umbilical cord blood, ES cell, iPS cell, bone marrow cell, or peripheral blood. That is, a population of only myeloid cells can be obtained stably and easily by adopting a method of immortalizing CD14-positive cells. Therefore, it is possible to stably provide myeloid cells that are closer to cells in the living body than conventional cells, and the cells are considered to be suitable for safety evaluation. Accordingly, the present invention provides safety evaluations such as evaluation of skin sensitizing property and/or pyrogenic property skin sensitizer and/or detection of pyrogen and the like by using immortalized myeloid cells. In addition, the present invention provides evaluation of an action of a sample on the function of immune cells by using immortalized myeloid cells.

Specifically, the present invention relates to the following invention.
(1) A method for evaluating the skin sensitizing property and/or the pyrogenic property of a test substance, comprising measuring an amount of IL-6 and/or IL-8 produced in a culture medium by culturing human immortalized myeloid cells in the presence of the aforementioned test substance.
(2) The method of (1), wherein the aforementioned human immortalized myeloid cell is a human immortalized monocyte or a dendritic cell prepared from the human immortalized monocyte.
(3) The method of (1) or (2), wherein a concentration of the aforementioned test substance during the culture of the aforementioned human immortalized myeloid cells includes at least 3 kinds of concentration between higher than 0 µg/mL and not more than 1000 µg/mL.
(4) The method of any one of (1) to (3), wherein a time of the aforementioned culture is at least one kind selected from 3 hr to 48 hr.
(5) The method of any one of (1) to (4), wherein the aforementioned production amount is measured by an immunological measurement method.
(6) The method of (5), wherein the aforementioned immunological measurement method is ELISA.
(7) The method of any one of (1) to (6), further comprising determining that the aforementioned test substance has skin sensitizing property and/or pyrogenic property when the aforementioned production amount is not less than 1.5 times the production amount of a negative control.
(8) A method for detecting a skin sensitizer and/or a pyrogen in a sample, comprising measuring an amount of IL-6 and/or IL-8 produced in a culture medium by culturing human immortalized myeloid cells in the presence of the aforementioned sample.
(9) The method of (8), wherein the aforementioned human immortalized myeloid cell is a human immortalized monocyte or a dendritic cell prepared from the human immortalized monocyte.
(10) The method of (8) or (9), wherein a concentration of the aforementioned sample during the culture of the aforementioned human immortalized myeloid cells includes at least 3 kinds of concentrations.
(11) The method of any one of (8) to (10), wherein time of the aforementioned culture is at least one kind selected from 3 hr to 48 hr.
(12) The method of any one of (8) to (11), wherein the aforementioned production amount is measured by an immunological measurement method.
(13) The method of (12), wherein the aforementioned immunological measurement method is ELISA.
(14) The method of any one of (8) to (13), further comprising determining that the aforementioned sample comprises a skin sensitizer and/or a pyrogen when the aforementioned production amount increased than the production amount of a negative control.
(15) The method of any one of (8) to (14), wherein the aforementioned skin sensitizer and/or pyrogen are/is lipopolysaccharide (LPS) and/or Staphylococcus aureus Cowan 1 (SAC).
(16) A method for evaluating an action of a sample on a function of immune cells, comprising measuring an amount of cytokine produced in a culture medium by culturing human immortalized myeloid cells in the presence of the aforementioned sample.
(17) The method of (16), wherein the aforementioned human immortalized myeloid cell is a human immortalized monocyte or a dendritic cell prepared from the human immortalized monocyte.
(18) The method of (16) or (17), wherein the aforementioned cytokine is selected from the group consisting of IL-1a, IL-1b, IL-1ra, IL-2, IL-2Ra, IL-3, IL-4, IL-5, IL-6, IL-7, IL-8, IL-9, IL-10, IL-12(p70), IL-12(p40), IL-13, IL-15, IL-16, IL-17A, IL-18, CTACK, Eotaxin, FGF basic, G-CSF, GM-CSF, GRO-α, HGF, IFN-α2, IFN-γ, IP-10, LIF, MCP-1(MCAF), MCP-3, M-CSF, MIF, MIG, MIP-1α, MIP-1β, β-NGF, PDGF-BB, RANTES, SCF, SCGF-β, SDF-1a, TNF-α, TNF-β, TRAIL, and VEGF-A.
(19) The method of any one of (16) to (18), wherein the aforementioned action on the function of immune cells is selected from an action to increase cytokine production from immune cells, an action to decrease cytokine production from immune cells, an inflammation-inducing action by immune cells, an inflammation-suppressing-inducing action by immune cells, an immunity activation-inducing action by immune cells, an immunity suppression-inducing action by immune cells, a mastocyte-stimulating action by immune cells, an IgE production-enhancing action by immune cells, an IgE production-suppressing action by immune cells, an IgA production-enhancing action by immune cells, a lymphocyte-proliferating and activating action by immune cells, an eosinophil production-increasing action by immune cells, a leukocyte migration-promoting action by immune cells, an apoptosis-inducing action by immune cells, an apoptosis-suppressing action by immune cells, a virus resistant-increasing action by immune cells, a cell proliferation-promoting action by immune cells, an angiogenesis inducing action by immune cells, a wound-healing action by immune cells, a hematopoietic cell proliferation-promoting action by immune cells, a progenitor cell differentiating action by immune cells, and a cell differentiation-inhibiting action by immune cells.
(20) The method of any one of (16) to (19), wherein a time of the aforementioned culture is 3 hr to 48 hr.
(21) The method of any one of (16) to (20), further comprising determining that the aforementioned sample has functionality when the aforementioned production amount of the aforementioned cytokine in the aforementioned culture medium obtained by culturing the aforementioned human immortalized myeloid cells in the presence of the aforementioned sample is not less than 2 times the production amount of cytokine in a culture medium obtained by culturing the aforementioned human immortalized myeloid cells in the presence of a negative control.
(22) The method of any one of (16) to (21), wherein the aforementioned sample is a functional beverage.
(23) A kit for use in the method of any one of (16) to (22), comprising a human immortalized monocyte or a dendritic cell prepared from the human immortalized monocyte.

### [Advantageous Effects of Invention]

According to the present invention, the research and evaluation of safety tests relating to the detection of skin sensitizing property and pyrogenic property of a substance, and the like become possible. For example, even for the research and evaluation of safety, which was difficult to study because stable evaluation was difficult with blood cell materials derived from human blood donation, precise evaluation with better sensitivity is possible using the present invention, and the research and evaluation of safety can be performed. According to the method of the present invention, moreover, safety evaluation of a drug can be performed with less error. Furthermore, due to the above-mentioned characteristics, the method of the present invention can also be used as a screening method for safety evaluation. In addition, due to the above-mentioned characteristics, the method of the present invention can also be used as a method for evaluating an action of a sample on the function of immune cells, and a screening method for a substance having an action on the function of immune cells.

### [Brief Description of Drawings]

[Fig. 1]

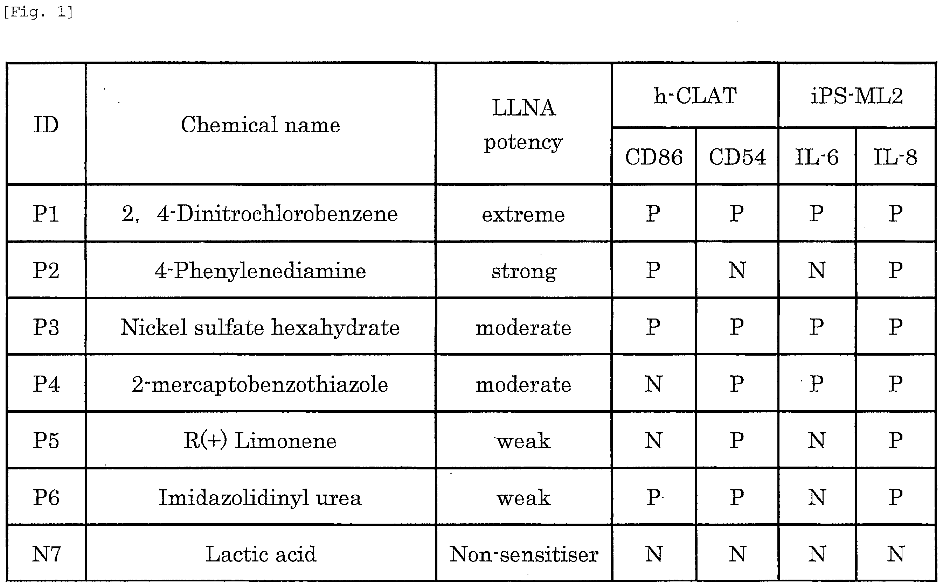

   Fig. 1 shows the evaluation results of the skin sensitizing property and/or the pyrogenic property of each test substance and the results of a conventional skin sensitization evaluation method.
[Fig. 2]

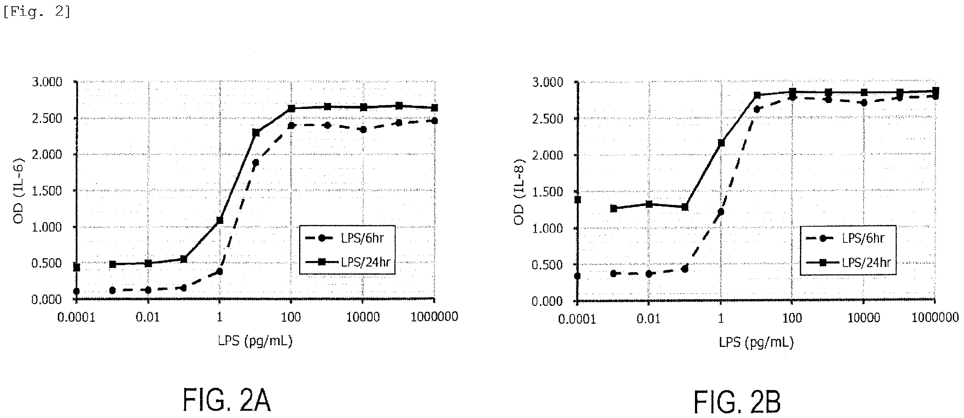

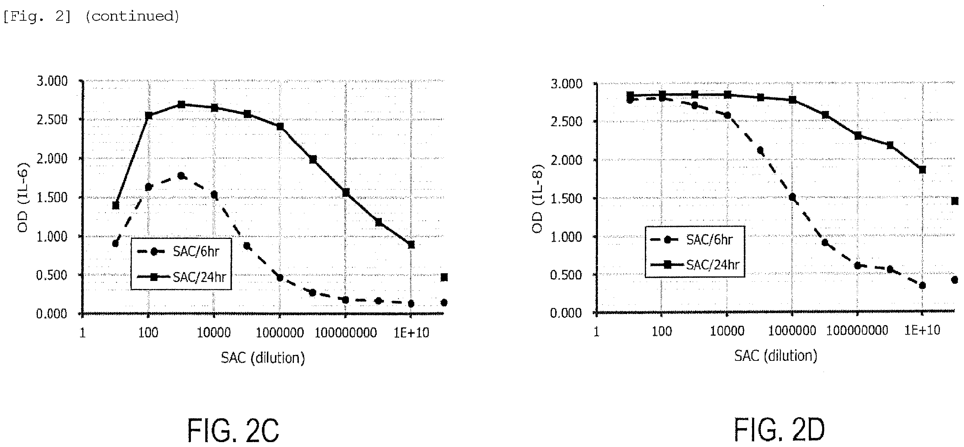

   Fig. 2A and Fig. 2B are graphs showing the production amounts as measured by ELISA of IL-6 and IL-8 in culture supernatants obtained by culturing iPS-ML2 for 6 hr and 24 hr in the presence of LPS at each concentration. The vertical axis of the graph shows the measured value of ELISA, and the horizontal axis of the graph shows the concentration of LPS. Fig. 2C and Fig. 2D are graphs showing the production amounts as measured by ELISA of IL-6 and IL-8 in culture supernatants obtained by culturing iPS-ML2 for 6 hr and 24 hr in the presence of SAC at each concentration. The vertical axis of the graph shows the measured value of ELISA, and the horizontal axis of the graph shows the dilution degree of SAC.
[Fig. 3]

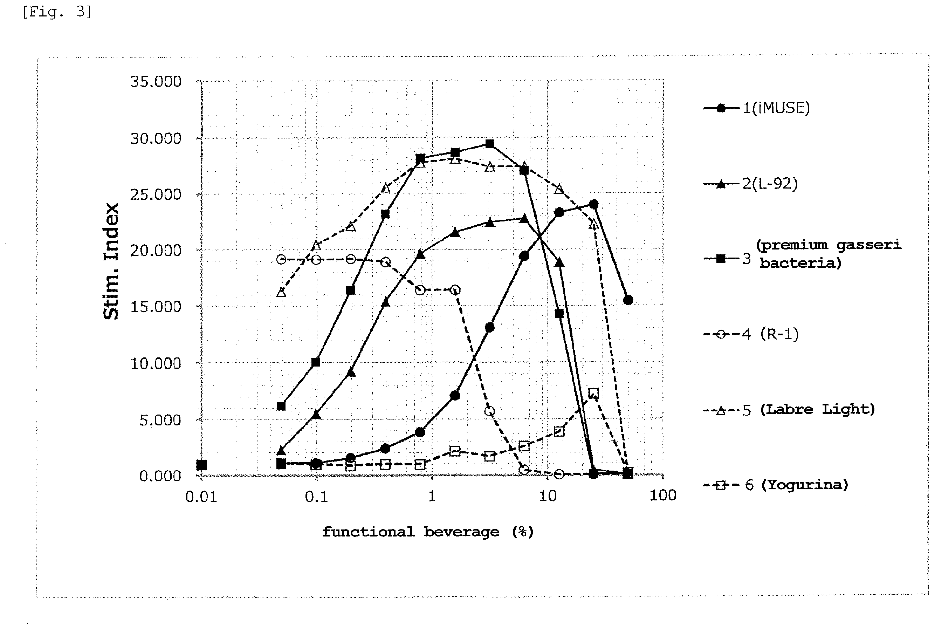

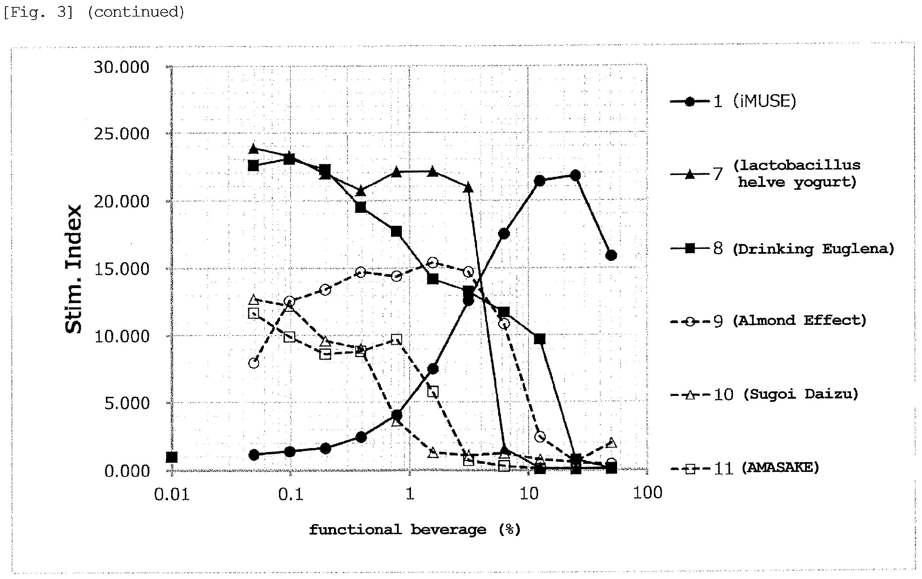

   Fig. 3 is a graph showing the production amount ratio (Stim. Index) obtained by measuring by ELISA the production amount of IL-6 in the culture supernatant obtained by culturing iPS-ML2 for 24 hr in the presence of functional beverages at each concentration, and dividing the measured value of the cell supernatant after culturing in the presence of each functional beverage by the measured value of the negative control. The vertical axis of the graph shows the production amount ratio, and the horizontal axis of the graph shows the concentration (%) of the functional beverage.

### [Description of Embodiments]

### <definition of terms>

In the present specification, the "skin sensitization" is one of the delayed hypersensitivity reactions, and refers to a phenomenon in which the skin is inflamed due to an excessive immune response caused by a substance.

In the present specification, the "substance" may be a monoatomic molecule, a compound, or the like.

In the present specification, the "pyrogen" refers to a substance that causes an increase in body temperature when taken into the body. Examples thereof include endotoxin, peptide glycan, exotoxin of gram-positive bacteria, virus, pathogenic bacterium, pathogenic fungi, lipopolysaccharide (LPS), Staphylococcus aureus Cowan 1 (SAC), and the like. Such increase in the body temperature may be caused by an immune response of a sample to which the substance was inoculated.

In the present specification, the "myeloid cell" is defined as a cell expressing a CD11b molecule or a CD33 molecule, and its origin is not particularly limited. Examples thereof include a myeloid cell derived from a pluripotent stem cell and a myeloid cell collected from a living body (e.g., peripheral blood). Specific examples of the myeloid cell include monocyte, dendritic cell, macrophage, and the like.

In the present specification, the "test substance" is not particularly limited as long as it is a substance for which skin sensitizing property and/or pyrogenic property is to be evaluated.

In the present specification, examples of the "sample" include body fluid (e.g., blood, tear, saliva, or urine) or tissue derived from human or animal, plant and extract thereof, food such as fruit, vegetable and the like, and extract thereof, foods such as processed food and beverage and the like, soap, shampoo, rinse and treatment, detergent, dye, fiber, cloth, cosmetic, pharmaceutical product, compound, mixture (e.g., food, food extract, plant extract), trace substance in atmosphere, exhaust gas, waste liquid, industrial waste, cell culture medium, cultured cell, medium for cell culture, additive for cell culture, cell preservative solution, food with health claims, supplement, and the like.

In the present specification, the "food with health claims" is a group of foods such as processed foods and beverages that are eaten for the purpose of maintaining or improving health, and includes food for specified health uses, food with nutrient function claims, and food with functional claims. In the present specification, the "functionality" refers to properties that are effective in maintaining and improving health (e.g., bioregulation, improvement of immunity, prevention of diseases, etc.). In the present specification, the "functional beverage" refers to a beverage containing a functional component (e.g., lactobacillus).

In the present specification, the "about" means a range of ±10%.

### <preparation method of immortalized monocyte>

In the present invention, monocytes to be a starting material can be obtained by a method of collecting from peripheral blood, a method of inducing differentiation from pluripotent stem cells, a method of inducing differentiation from other somatic cells such as fibroblasts, and the like.

When monocytes are collected from peripheral blood, a method for separating and preparing cells expressing the CD14 molecule in human peripheral blood is known. For example, human peripheral blood is gently diluted with an equal amount of saline, phosphate buffered saline, Hanks' buffered solution, or the like. The diluted blood is gently laminated on Ficoll (registered trade mark) (GE Healthcare) in a centrifuge tube, and centrifuged at 15 to 30°C, 500 to 1000×G for 20 min. A white band-like layer between yellowish plasma and transparent Ficoll is collected as a peripheral blood mononuclear cell (PBMC) fraction consisting of lymphocytes and monocytes. The collected peripheral blood mononuclear cell fraction may be further washed and used if necessary. Monocytes can be obtained by further recovering cells expressing the CD14 molecule from the recovered PBMC fraction. For example, monocytes can be separated and recovered by bringing a solid phase with an anti-CD14 antibody bound thereto and PBMC into contact with each other to allow binding of the monocytes to the solid phase, and removing the unbound cells by washing. For example, a method using magnetic beads as such solid phase and the like are known (Dynabeads (registered trade mark) CD14 (Thermo Fisher Scientific Inc.) and the like). Monocytes can also be obtained by directly contacting the peripheral blood with the solid phase to which the anti-CD14 antibody is bound, without separating PBMC from the peripheral blood.

When monocytes are obtained by differentiation from pluripotent cells, a method for inducing monocytes from pluripotent cells is known. In the present specification, the "pluripotent stem cell" means a cell having pluripotency and self-replication ability. In the present specification, the "pluripotency" is synonymous with multilineage potential and means a state of cell capable of differentiating into cells of multiple lineages by differentiation. In the present specification, the pluripotency includes a state in which differentiation into all types of cells constituting a living body is possible (totipotency), a state in which differentiation into all types of cells except extraembryonic tissue is possible (pluripotency), a state in which differentiation into cells belonging to some cell lineages is possible (multipotency), and a state in which differentiation into one type of cell is possible (unipotency). Therefore, the "pluripotent stem cell" in the present specification includes stem cell, embryonic stem (ES) cell, embryonic stem cells derived from a clone embryo obtained by nucleus transplantation ("ntES cell"), germ stem cell ("GS cell"), embryonic germ cell ("EG cell"), induced pluripotent stem (iPS) cell, and hematopoietic stem cell. As to whether or not a cell is a pluripotent stem cell, for example, when a test cell forms an embryoid (embryoid body) in an ex-vivo culture system, or when it is differentiated into a desired cell after being cultured (differentiation treatment) under differentiation-inducing conditions, the cell can be determined to be a pluripotent stem cell. Methods for obtaining pluripotent stem cells are known. For example, it has been reported that an iPS cell can be obtained by introducing not less than 3 kinds of reprogramming genes (Oct3/4, Sox2, Klf4, c-Myc, L-Myc, Nanog, Lin28, Esrrb, Glis1, E-cadherin, shp53, UTX, H1foo, and the like) into somatic cells.

Induction of differentiation of pluripotent stem cells into monocytes can be performed by contacting, for example, macrophage colony stimulating factor (M-CSF) and interleukin 3 (IL-3) (Fernadndo O. Martinez et al., Experimental Hematology (2008); 36:1167-1175), or IFN-γ or PMA (Annabelle Grolleau et al., J Immunol 1999; 162:3491-3497) with pluripotent stem cells. Only the cells expressing CD14 may be recovered from the monocytes after differentiation induction and purified by the aforementioned method as necessary.

As a method for inducing differentiation of monocytes from other somatic cells such as fibroblast and the like, the method described in Takahiro Suzuki et al., "Reconstruction of Monocyte Transcriptional Regulatory Network Accompanies Monocytic Functions in Human Fibroblasts", PLoS One (2012) doi:10.1371/journal.pone.0033474 can be mentioned.

Proliferation potency can be added to the obtained monocyte while maintaining the function of monocyte, by introducing at least one gene selected from BMI1 gene, EZH2 gene, MDM2 gene, MDM4 gene, HIF1A gene, BCL2 gene, LYL1 gene, and cMYC gene. Preferred is a combination of at least one gene selected from BMI1 gene, EZH2 gene, MDM2 gene, MDM4 gene, and HIF1A gene, and cMYC gene; a combination of BMI1 gene and BCL2 gene, and cMYC gene; or a combination of LYL1 gene and cMYC gene. Introduction of these genes into monocytes can be performed in consideration of the descriptions in WO2012/043651 and JP-A-2017-131136. For example, these genes can be introduced by infecting monocytes with a lentiviral vector carrying them.

### <preparation method of dendritic cell>

Induction of dendritic cells from immortalized monocytes can be performed by culturing monocytes in the presence of, for example, 200 IU/ml GM-CSF and 200 IU/ml IL-4 according to previous reports (Francoise Chapuis et al., Eur J Immunol.(1997) 27(2):431-41.; Marc Dauer et al., J Immunol (2003) 170(8):4069-4076.; Figdor CG et al., Nat Med. (2004) 10(5):475-80; Helmut Jonuleit et al., Eur J Immunol. (1997) 27(12):3135-42).

### <evaluation of skin sensitizing property and/or pyrogenic property of a substance by using immortalized monocyte or dendritic cell>

Evaluation of skin sensitizing property and/or pyrogenic property of a substance by using immortalized monocyte or dendritic cell can be performed by the steps shown below.

In the first step, cells for evaluation are prepared. As the cells to be used for evaluation, the aforementioned immortalized monocytes or dendritic cells are used. Examples of the medium to be used for culturing during the evaluation and/or before or after evaluation include IMDM solution, RPMI solution, α-MEM solution, MEM medium, and DMEM solution. As an additive, human or bovine plasma protein fraction, bovine fetal serum or human serum, saccharide such as glucose, D-mannitol or the like, amino acid, nucleic acid base such as adenine or the like, sodium hydrogenphosphate, A-tocopherol, linoleic acid, cholesterol, sodium selenite, human holotransferrin, human insulin, ethanolamine, 2-mercaptoethanol, G-CSF, GM-CSF, sodium hydrogen carbonate, methylcellulose or the like may be contained. Where necessary, antibiotic such as gentamicin, ampicillin, penicillin, streptomycin or the like; inorganic salt; buffering agent such as HEPES, phosphate buffering agent, acetate buffering agent, or the like may be added. In a specific example, an α-MEM (10% bovine fetal serum) medium supplemented with non-essential amino acid, a culture medium obtained by adding 2 or 5% HPL (Human platelet lysate, human platelet-derived supplement) to α-MEM, or RPMI1640 medium (SIGMA) containing hypoxanthine and HEPES (SIGMA), and supplemented with 10% bovine fetal serum, sodium hydrogen carbonate, 100 unit/mL penicillin, and 100 mg/mL streptomycin can be used.

Immortalized monocytes or dendritic cells are seeded in the aforementioned medium, and the cells are passaged a given number of times. The number of passages may be, for example, once, twice, three times, four times, or five times or more. Then, the cells after passage are washed a given number of times (e.g., twice, three times, or four times or more), prepared to a predetermined concentration with a medium used for the evaluation, and used for the evaluation. The cell concentration can be determined according to the protocol of the immunological measurement method described later.

As the second step, the aforementioned immortalized monocytes or dendritic cells are cultured in the presence of a test substance. A test substance is dissolved in a solution and used for the evaluation. As the solution used for dissolving a test substance, ethanol, DMSO, water, PBS, and the like may be used. A test substance is diluted such that it contacts the cells at a previously-determined final concentration, and used for the evaluation. The previously-determined final concentration is one or more, preferably at least 3, kinds of concentration between higher than 0 µg/mL and not more than 1000 µg/mL prepared. The adjusted concentration may be 3 kinds, or 4 kinds, or 5 kinds, or not less than 6 kinds. A test substance may be diluted using the aforementioned medium.

As the culture time of immortalized monocytes or dendritic cells, one kind of time may be set or two kinds of time may be set. In addition, three or more kinds of time may be set. For example, the culture time may be set to any time selected between 3 and 48 hr, preferably 6 and 24 hr. In addition, the temperature during culture may be about 37°C, and the carbon dioxide concentration during culture may be about 5%.

In the third step, IL-6 and/or IL-8 in the culture medium of immortalized monocytes or dendritic cells after culturing are/is measured. Examples of the culture medium include culture supernatant. The measurement can be performed using a known immunological measurement method. The immunological measurement method may be, for example, ELISA (Enzyme-Linked Immuno Sorbent Assay), immuno-chromatography, radioimmunoassay, or Western blot. These immunological measurements may be performed using a commercially available kit.

Whether or not a test substance has skin sensitizing property and/or pyrogenic property is determined based on the measured production amount of IL-6 and/or IL-8. To be specific, when the production amount of IL-6 and/or IL-8 in a culture medium of immortalized monocytes or dendritic cells obtained by culturing for a given time in the presence of a test substance increased as compared with the negative control, the test substance is determined to have skin sensitizing property and/or pyrogenic property. For example, the increase may be set with a certain rate of increase relative to the negative control as a threshold value, and when the production amount exceeds the rate of increase, the test substance may be determined to have skin sensitizing property and/or pyrogenic property. Such increase rate can be set to a value between 1.2 and 2.0 times, for example, 1.5 times. When the evaluation is performed using multiple concentrations of a test substance, the test substance may be determined to have skin sensitizing property and/or pyrogenic property when the production amount of IL-6 and/or IL-8 in the culture medium obtained by culturing in the presence of at least one kind of concentration of the test substance, among the prepared multiple concentrations, increased as compared with the negative control. When the evaluation is performed with multiple culture times set in the presence of a test substance, the test substance may be determined to have skin sensitizing property and/or pyrogenic property when the production amount of IL-6 and/or IL-8 in the culture medium obtained by culturing in the presence of any concentration of the test substance in at least one kind of culture time, among the multiple culture times set, increased as compared with the negative control.

As the negative control, a lysate for dissolving the test substance may be used, or the medium used for culturing immortalized monocytes or dendritic cells may be used.

### <detection of skin sensitizer and/or pyrogen in a sample by using immortalized monocyte or dendritic cell>

Detection of skin sensitizer and/or pyrogen in a sample by using immortalized monocyte or dendritic cell is performed by measuring the production amount of IL-6 and/or IL-8 in the culture medium obtained by culturing immortalized monocytes or dendritic cells in the presence of a sample.

Since the basic method for the detection method of the skin sensitizer and/or pyrogen in the sample is the same as in the aforementioned <evaluation of skin sensitizing property and/or pyrogenic property of a substance by using immortalized monocyte or dendritic cell>, only the differences are described in detail in this section.

A skin sensitizer and/or a pyrogen (e.g., LPS and/or SAC) are/is dissolved in a solution and used for the evaluation. As such solution, ethanol, DMSO, water, PBS, and the like may be used. The skin sensitizer and/or pyrogen are/is diluted such that they contact the cells at a previously-determined final concentration, and used for the evaluation.

As the previously-determined final concentration, for example, one or more kinds, preferably at least 3 kinds, of concentrations are prepared between higher than 0.0001 pg/mL and not more than 1000000 pg/mL in the case of LPS, and a dilution degree between 10¹-fold and 10¹⁰-fold in the case of SAC. Three kinds, or four kinds, or five kinds, or not less than six kinds, of the concentration may be adjusted. Dilution of the skin sensitizer and/or pyrogen may be performed using the medium described in the section of <evaluation of skin sensitizing property and/or pyrogenic property of a substance by using immortalized monocyte or dendritic cell>. Such skin sensitizer and/or pyrogen may be used as a positive control in the detection of the skin sensitizer and/or pyrogen in a sample.

The sample used for evaluation may be diluted at any dilution ratio. Any one kind of dilution ratio may be selected from 1.2 times to 100 times. Two, three, or four or more kinds of dilution ratios may be selected. The sample may be diluted with water, physiological saline, the medium used for culturing immortalized monocytes or dendritic cells, or the like.

The detection of a skin sensitizer and/or a pyrogen in a sample is determined based on the measured production amount of IL-6 and/or IL-8. To be specific, when the production amount of IL-6 and/or IL-8 in a culture medium of immortalized monocytes or dendritic cells that were cultured for a given time in the presence of a sample increased as compared with the negative control, the sample is determined to contain a skin sensitizer and/or a pyrogen. When the evaluation is performed with multiple culture times set in the presence of a sample, the sample may be determined to contain a skin sensitizer and/or a pyrogen when the production amount of IL-6 and/or IL-8 in a culture medium in at least one kind of culture time, among the multiple culture times set, increased as compared with the negative control.

When the sample is diluted at multiple dilution ratios and the evaluation is performed, the sample is determined to contain a skin sensitizer and/or a pyrogen when the production amount of IL-6 and/or IL-8 in a culture medium of the cells obtained by culturing in the presence of at least one kind of dilution ratio of the sample, among the samples diluted at multiple dilution ratios, increased as compared with the negative control.

As the negative control, a lysate for dissolving a skin sensitizer and/or a pyrogen, physiological saline, the medium used for culturing immortalized monocytes or dendritic cells, or the like may be used.

### <evaluation of function of a sample on function of immune cells by using immortalized monocyte or dendritic cell>

The function of immune cells can be measured by measuring various cytokines produced by immortalized monocyte cells or dendritic cells. Therefore, the present invention can evaluate the action of a sample on the function of immune cells by utilizing the measurement of various cytokines produced by such immortalized monocyte cells or dendritic cells. Cytokines produced by immortalized monocyte cells or dendritic cells include interleukin, chemokine, tumor necrosis factor superfamily, interferon, growth factor, hematopoietic factor, colony stimulating factor, and the like.

Examples of the interleukin include IL-1α, IL-1β, IL-1Ra, IL-2, IL-2Ra, IL-3, IL-4, IL-5, IL-6, IL-7, IL-8, IL-9, IL-10, IL-11, IL-12 (p70), IL-12 (p40), IL-13, IL-15, IL-16, IL-17A, IL-18, and the like.

Among these interleukins, for example, IL-1α, IL-1β, IL-1Ra, IL-6, IL-8, IL-12, IL-17A, and IL-18 are known as inflammatory cytokines, and involved in skin inflammation or elevation of body temperature. On the other hand, IL-4 and IL-10 are known as anti-inflammatory cytokines. Thus, in one embodiment, the function of immune cells may be an inflammation-inducing action such as skin inflammation, elevation of body temperature or the like, or an inflammation suppression-inducing action.

In addition, interleukin is known to have various physiological activities related to health. IL-4 and IL-13 are known to induce IgE production, and IL-9 is known to stimulate mastocyte proliferation. On the other hand, IL-12 is known to have an IgE production suppressive action. Therefore, in one embodiment, the function of immune cells may be a mastocyte stimulating action, an IgE production enhancing action, or an IgE production suppressive action.

Also, IL-5 and IL-6 are known to enhance production of IgA (Jpn J. Lactic Acid Bact, 2007, 18(2), 54-57). Therefore, in one embodiment, the function of immune cells may be an IgA production-enhancing action.

IL-2 and IL-2Ra are known to have an action to proliferate and activate T cells, B cells, and natural killer (NK) cells. IL-7 is known to be involved in the survival, differentiation, and the like of B cells, T cells, and NK cells. IL-15 is known to have an action to proliferate and activate T cells and B cells. Thus, in one embodiment, the function of immune cells may be a lymphocyte (i.e., T cell, B cell, or NK cell)-proliferating and activating action.

IL-5 is known to have an action to increase production of eosinophils. Thus, in one embodiment, the function of immune cells may be an action to increase production of eosinophils.

IL-17A is known to induce production of inflammatory cytokines, IL-18 is known to induce interferon γ production together with IL-2, and IL-18 alone is known to induce IL-4 production. Thus, in one embodiment, the function of immune cells may be an action to increase cytokine production.

Examples of chemokine include IL-8, CTACK, Eotaxin, GRO-α, IP-10, MCP-1, MCP-3, MIG, MIP-1α, MIP-1β, RANTES, SDF-1α, and the like. These chemokines cause the migration of leukocytes from blood vessels into inflammatory tissues.

For example, IL-8 is considered to have a protective effect (pathogenic bacteria elimination effect) on infectious diseases through activation of neutrophils, and the like (The Japanese Journal of Antibiotics, 2013, 66-6, 305-310). CTACK is a skin-specific chemokine and is known to be involved in increased eosinophil production. Eotaxin is known to have strong eosinophil migration activity. GRO-α is known to have a cell division function and be a neutrophil activator. IP-10 is known to cause migration to monocyte, macrophage, and the like. MCP-1 (MCAF) and MCP-3 are known to cause migration of monocyte and macrophage. MIG is known to migrate T cells, a part of macrophages, and the like to the site of inflammation. MIP-1α is known to cause migration of monocyte and basophil. MIP-1β is known to cause migration of monocyte and eosinophil. RANTES is known to cause migration of monocyte, eosinophil, and basophil. SDF-1α is a strong chemotactic factor for lymphocytes, and is known to supplement lymphocytes to newly-formed blood vessels and be involved in angiogenesis in living organisms.

Among the above-mentioned interleukins, IL-16 is also known to have an action of migrating CD4 positive T cells.

Therefore, in one embodiment, the function of immune cells may be a leukocyte (i.e., neutrophil, eosinophil, basophil, lymphocyte, monocyte, or macrophage) migration promoting action, or a preventive action against infections.

Examples of the tumor necrosis factor superfamily include Tumor Necrosis Factor (TNF)-α, TNF-β, and TNF related apoptosis-inducing ligand (TRAIL). TNF-α and TNF-β are known to be involved in prevention of infection and an antitumor action by the expression of cell adhesion molecules, the induction of apoptosis, and the promotion of inflammation mediators (IL-1, IL-6, prostaglandin E2, etc.). TRAIL is known to induce apoptosis of cancer cells.

Therefore, in one embodiment, the function of immune cells may be an apoptosis inducing action.

Examples of the interferon include IFN-α (1, 2, 4, 5, 6, 7, 8, 10, 13, 14, 16, 17, 21), IFN-β, IFN-γ, and the like. Interferon is known to increase the virus resistance of cells by suppressing virus replication.

Therefore, in one embodiment, the function of immune cells may be a virus resistance increasing action.

Examples of the growth factor include basic fibroblast growth factor (FGF basic), hepatocyte growth factor (HGF), nerve growth factor (NGF), platelet derived growth factor (PDGF), stem cell growth factors (SCGF) α, β, vascular endothelial growth factors (VEGF) A-E, epidermal growth factor (EGF), and the like.

FGF basic is known to play an important role not only in promoting cell division but also in angiogenesis and wound healing. HGF is known to play an important role in promoting cell division, angiogenesis, tissue repair, and the like. NGF is known to have an action of extending nerve axons and promoting the synthesis of neurotransmitters, an action of maintaining nerve cells, an action of repairing cell damage, and the like. PDGF is mainly involved in the regulation of migration, proliferation and the like of mesenchymal cells, and is known to be involved in angiogenesis and wound healing. SCGFα and β are known to be mainly involved in the proliferation of hematopoietic cells. VEGF A-E are known to be involved in the proliferation of vascular endothelial cells, angiogenesis, and the like. EGF is known to be also involved in the proliferation of epithelial cells and wound healing.

Therefore, in one embodiment, the function of immune cells may be a cell proliferation promoting action, an angiogenesis inducing action, or a wound healing action.

Examples of the hematopoietic factor include stem cell factor (SCF) and the like. SCF is a hematopoietic growth factor that functions by signal transduction via a c-kit receptor, and is known to be an important factor for the survival, proliferation, and differentiation of hematopoietic cells.

Among the aforementioned interleukins, IL-3 is also known to have an action as a hematopoietic factor in support of the proliferation of immature blood cells.

Therefore, in one embodiment, the function of immune cells may be a hematopoietic cell proliferation promoting action.

Examples of the colony stimulating factor include granulocyte colony-stimulating factor (G-CSF), granulocyte macrophage colony-stimulating factor (GM-CSF), macrophage colony-stimulating factor (M-CSF), and the like. GM-CSF is known to act on granulocyte-macrophage progenitor cells to cause differentiation into granulocyte progenitor cells and macrophage progenitor cells. G-CSF is known to act on granulocyte progenitor cells to cause differentiation into neutrophils, and M-CSF is known to act on macrophage progenitor cells to cause differentiation into macrophages, thus exhibiting various physiological actions (the journal of the Japanese society of internal medicine 1996, 85, 844-849).

Therefore, in one embodiment, the function of immune cells may be a progenitor cell differentiation action.

As other cytokine, a leukemia inhibitory factor (LIF) is a cytokine of an IL-6 family that inhibits cell differentiation and affects cell growth. Therefore, in one embodiment, the function of immune cells may be a cell differentiation inhibitory action.

Macrophage migration inhibitory factor (MIF) induces cytokines such as TNF-α and IL-1β and aggravates inflammatory symptoms such as allergy and the like, while showing an action to regulate the induction of expression of the cancer suppressor gene p53, and is known to have an action to suppress apoptosis induced by p53 (Carcinogenesis. 2009, 30, 1597-605). Therefore, in one embodiment, the function of immune cells may be an action to regulate the induction of p53 expression, or an apoptosis suppressive action.

The functions of immune cells are not limited to those described above, and may be, for example, an action to decrease the amount of cytokine production from immune cells, an immunity activation-inducing action by immune cells, or an immunity suppression-inducing action by immune cells.

The evaluation method of the present invention can be performed by measuring the production amount of cytokine in the culture medium obtained by culturing immortalized monocyte cells or dendritic cells in the presence of a sample. In this method, evaluation of an action on the function of immune cells can be performed using the production amount of cytokine as an index. A specific evaluation method is described in detail in the following.

The evaluation of an action of a sample on the function of immune cells can be performed according to the aforementioned <evaluation of skin sensitizing property and/or pyrogenic property of a substance by using immortalized monocyte or dendritic cell>.

In the evaluation method of the present invention, the cytokines to be subjected to the measurement of production amount are preferably IL-6, IL-8, IL-12 (p40), MIP-1α, MIP-1β, GRO-α, TNF-α, MCP-1(MCAF), IFN-γ, and G-CSF.

The sample can be dissolved, suspended or diluted with a solution, suspension or dilution liquid, or used as is for evaluation. As such solution, organic solvent (ethanol, DMSO, etc.), water, medium (the medium described in the section of <evaluation of skin sensitizing property and/or pyrogenic property of a substance by using immortalized monocyte or dendritic cell> and the like), buffer (PBS and the like) may be used. As the sample, a commercially available product (e.g., functional beverage or pharmaceutical product) may be used.

In addition, a sample may be dissolved or diluted such that it contacts the cells at a previously-determined final concentration or dilution rate, and used for the evaluation. The final concentration to be adjusted may be one or more, preferably at least 3, kinds of concentration between higher than 0% and not more than 50%. The kind of the final concentration or dilution rate may be 3 kinds, or 4 kinds, or 5 kinds, or not less than 6 kinds.

The production amount of cytokine in the culture medium of immortalized monocytes or dendritic cells after culturing is measured. As the culture medium to be measured, culture supernatant is generally used; however, a cell homogenate fluid may also be used as necessary. The measurement can be performed using a known immunological measurement method. The immunological measurement method may be, for example, ELISA (Enzyme-Linked Immuno Sorbent Assay), immuno-chromatography, radioimmunoassay, Western blot, or multiplex assay. These immunological measurements may be performed using a commercially available kit.

Whether or not a sample has an action on the function of immune cells is determined based on the measured production amount of cytokine. To be specific, when the production amount of IL-6 and/or IL-8 in a culture medium of immortalized monocytes or dendritic cells obtained by culturing for a given time in the presence of a sample increased as compared with the negative control, the sample is determined to have an action on the function of immune cells. In this case, for example, the increase may be set with a certain rate of increase relative to the negative control as a threshold value, and when the production amount exceeds the rate of increase, the sample may be determined to have functionality. Such increase rate may be not less than 1.2 fold, specifically 2 fold, 3 fold, 5 fold, or not less than 10 fold.

When the evaluation is performed using multiple concentrations of a sample, the sample may be determined to have an action on the function of immune cells when the production amount of cytokine in the culture medium obtained by culturing in the presence of at least one kind of concentration of the sample, among the prepared multiple concentrations, increased as compared with the negative control. In the case of evaluation while setting multiple culture times in the presence of a sample, when the production amount of cytokine in the culture medium of the cells obtained by culturing in the presence of a sample at any concentration in at least one kind of culture time, among the multiple culture times set, increased as compared with the negative control, the sample may be determined to have an action on the function of immune cells.

As the negative control, for example, the medium used for culturing immortalized monocytes or dendritic cells may be used.

### <evaluation kit>

The present invention relates to a kit containing human immortalized monocytes or dendritic cells prepared from the human immortalized monocytes, which is used for the evaluation of the skin sensitizing property and/or the pyrogenic property of a substance, the detection of pyrogen in a sample, and/or the evaluation of an action on the function of immune cells. The kit may contain packages and instructions in addition to the aforementioned monocytes or dendritic cells. It may also contain culture medium, culture additive, laboratory equipment, anti-IL-6 antibody and/or anti-IL-8 antibody, any anti-cytokine antibody, and any standard substance. As the culture medium, the medium described in the section of <evaluation of skin sensitizing property and/or pyrogenic property of a substance by using immortalized monocyte or dendritic cell> can be used.

### [Example]

The present invention is explained in more detail in the following based on Examples; however, the present invention is not limited by the following Examples. All documents cited throughout this application are directly incorporated herein by reference.

### (Example 1) Production of immortalized monocyte derived from human peripheral blood and production of dendritic cell (Production of immortalized monocyte derived from human peripheral blood)

Immortalized monocyte derived from human peripheral blood was produced by reference to previous reports (WO2012/043651 and JP-A-2017-131136). Specifically, a CD14-positive fraction was taken out from human peripheral blood, and the genes reported with respect to the production of human immortalized monocytes (c-MYC, BMI1, and BCL-2) were introduced according to the previous reports to prepare the monocytes. The immortalized monocyte strain was cultured in α-MEM medium containing 20% FBS (Cytiva Inc., Cat#: SH30088.03), 50 ng/mL M-CSF (Peprotech Inc., Cat#: AF-300-25), and 50 ng/mL GM-CSF (Peprotech Inc., Cat#: 300-03), acquired as proliferative cells 3 to 5 weeks after the start of culture, and preserved under liquid nitrogen using a commercially available cell preservation solution, for example, serum-free cell preservation solution BAMBANKER (GCLTEC Inc., Cat#: CS-02-001).

### (confirmation of prepared cells)

The measurement of surface markers of the prepared cells, observation of the morphology by Giemsa staining, and the like were performed. The surface markers were measured using a CD11 antibody and a CD14 antibody (both Biolegend Inc.) and a flow cytometer CyFlowspace of Sysmex Inc. Since the expression of CD11 and CD14 could be confirmed by the measurement, it was confirmed that the prepared cells expressed the monocyte-like marker. As for staining, a commercially available staining solution (Sigma) was used to perform Giemsa staining or May-Giemsa staining based on a known staining method, and the measurement was performed with a high-magnification microscope (400-1000x).

### (preparation of dendritic cell from immortalized monocyte)

The preserved immortalized monocyte strain was cultured in α-MEM medium (containing 20% FBS, 50 ng/mL M-CSF, and 50 ng/mL GM-CSF) in a 5% CO₂ incubator at 37°C, and maintained and prepared by exchanging the medium once every 3 days. Dendritic cells were prepared by culturing immortalized monocytes for 3 days in the presence of 50 ng/mL M-CSF, 50 ng/mL GM-CSF, and 100 ng/mL IL-4 to cause differentiation into dendritic cells.

### (Example 2) (Production of immortalized monocyte derived from human induced pluripotent stem cell)

Immortalized monocytes derived from human induced pluripotent stem (iPS) cells were produced by reference to previous reports (WO2012/043651, JP-A-2017-131136 and JP-A-2018-171005). Specifically, undifferentiated iPS cells (distributed from the Center for iPS Cell Research and Application, Kyoto University) were inoculated on a 10 cm diameter dish (AGC TECHNO GLASS Co., Ltd., Cat#:1012-100) or 6-well plate (Corning Inc., Cat#: 3516) coated with laminin 511 (iMatrix-511-E8, Nippi, Inc., Cat#: 892-012) as a basement membrane in advance, and differentiation-inducing culture was started using a culture medium obtained by adding 20% FBS (Cytiva Inc., Cat#:SH30088.03) to α-MEM medium (Fuji film Wako Pure Chemical Industries, Ltd., Cat#: 137-17215) (hereinafter referred to as α-MEM medium (containing 20% FBS)) differentiation induction culture. Thereafter, the culture was continued while exchanging the α-MEM medium (containing 20% FBS) once every 3 days. At 14 to 21 days after the start of differentiation induction, equal amounts of TrypLE^{™} Select (containing 1 mM ethylenediaminetetraacetic acid (EDTA)) (GIBCO Inc., Cat#: A12859-01) and 0.5 mM EDTA/PBS (Nacalai Tesque INC., Cat#: 13567-84) were mixed, and the cells were treated (37°C, 60 min) with the prepared solution (final concentration 0.75 mM EDTA). The cells were dissociated and collected, and a cell suspension was prepared by a pipetting operation. Subsequently, the cell suspension collected from one dish with a diameter of 10 cm or one well on a 6-well plate was suspended in 10 mL of D-MEM medium (Fujifilm Wako Pure Chemical Industries, Ltd., Cat#: 044-29765) (containing 10% FBS (Cytiva Inc., Cat#: SH30088.03)), and seeded on two dishes or one dish with a diameter of 10 cm without feeder cells, without laminin coat, and left standing in a 5% CO₂ incubator at 37°C. After about 16 hr, the cells that did not adhere to the dish were collected and passed through a 100-micrometer mesh (cell strainer manufactured by BD Falcon) to obtain a cell suspension after removal of coagulated cell aggregates.

The cell suspension after removal of the coagulated cell aggregates obtained above was seeded in α-MEM medium (containing 20% FBS, 100 ng/mL M-CSF (Peprotech Inc., Cat#: AF-300-25), and 100 ng/mL GM-CSF (Peprotech Inc., Cat#: 300-03)) and cultured in a T25 flask (CellSeed Inc., HydroCell, Cat#: CSF025, or Thermo Fisher Scientific Inc., Cat#: 169900). Thereafter, about 3 to 9 days later, floating or weakly adhesive cells appeared, and it was observed that the number of cells gradually increased. The floating cells (iPS-MC) were collected, and the expression of the leukocyte marker molecule CD45 and the myeloid cell marker CD11b or CD33 was confirmed using a flow cytometer (not shown).

Using a third-generation lentivirus vector (SignaGen Inc.) which is a strain deficient in human immunodeficiency virus type 1 (HIV-I) proliferation potential, etc., and whose promoter for protein expression is EF1a, a gene (Myc, Myb, Hob8, TLX1, E2A-pbxl, MLL, Ljx2, RARA, Hoxa9, Notch1, v-raf/v-myc, MYST3-NCOA2, Evil, HOXB6, HOXB4, β-catenin, Id1) reported for the production of mouse immortalized hematopoietic stem cells, or a gene (at least one from c-MYC and BMI1, EZH2, MDM2, MDM4, HIFIA) reported for the production of human immortalized monocytes was introduced into the floating cells (iPS-MC) obtained above to produce immortalized monocytes derived from human induced pluripotent stem cells. The immortalized monocyte strain was cultured in α-MEM medium containing 20% FBS, 50 ng or 50-100 ng/mL M-CSF, and 50 ng or 50-100 ng/mL GM-CSF in a 24-well plate and acquired as proliferative cells 3 to 5 weeks after the start of culture. The immortalized monocyte cells derived from human induced pluripotent stem cells were preserved under liquid nitrogen using a commercially available cell preservation solution, for example, a serum-free cell preservation solution BAMBANKER (GCLTEC Inc., Cat#: CS-02-001).

### (Example 3) Production of immortalized monocyte derived from human induced pluripotent stem cell expressing M-CSF and GM-CSF

Immortalized monocyte derived from human induced pluripotent stem cell expressing M-CSF and GM-CSF were prepared by reference to a previous report (JP-A- 2018-171005). Specifically, using a third-generation lentivirus vector (SignaGen Inc.) which is a strain deficient in human immunodeficiency virus type 1 (HIV-I) proliferation potential, etc., and whose promoter for protein expression is EF1a, and an expression vector for human M-CSF gene, and an expression vector for human GM-CSF gene were simultaneously introduced into the immortalized monocytes derived from human induced pluripotent stem cells obtained in Example 2. From 3 days after gene transfer, the cells were cultured in α-MEM (containing 20% FBS) medium not containing both human M-CSF and GM-CSF, acquired as proliferative cells 3 to 5 weeks after the start of culture, and preserved under liquid nitrogen using a commercially available cell preservation solution. The human immortalized monocytes prepared in Example 3 are hereafter to be referred to as iPS-ML2.

### (Example 4) Evaluation test of skin sensitizing property and/or pyrogenic property of substance by using immortalized monocyte strain

The evaluation test of skin sensitizing property and/or pyrogenic property was performed according to the following procedures. In the test, iPS-ML2 was used. Cryopreserved iPS-ML2 was thawed at room temperature, washed, seeded in T-25 (Thermo Fisher Scientific Inc) flask containing 10 mL of a medium (10% FCS-containing α-MEM (GIBCO)), passaged not less than 3 times, and subjected to the test.
1. The iPS-ML2 floating in the T-25 flask was transferred to a 15 mL tube. Thereafter, centrifugation was performed at room temperature, 300×G for 5 min, and the supernatant was discarded.
2. To the pellets of iPS-ML2 was added 10 mL of PBS (Fujifilm Wako Pure Chemical Industries, Ltd.), the mixture was centrifuged at room temperature, 300×G for 5 min, and the supernatant was discarded. This operation was repeated once more.
3. The iPS-ML2 after washing was suspended in a medium (10% FCS-containing α-MEM (GIBCO)).
4. Cell number was counted using trypan blue.
5. The iPS-ML2 was seeded to the cell number of 1×10⁴/100 µL per each well of 96-well flat plate (Coring).
6. As a test substance, a compound shown in the following Table 1 was dissolved in a solution, and this solution was adjusted with a medium (10% FCS-containing α-MEM (GIBCO)) to a concentration of 400 µg/mL.
7. The 400 µg/mL test substance was diluted 2-fold with a medium (10% FCS-containing α-MEM (GIBCO)), and a total of 11 concentrations were prepared.
8. The test substance at each concentration was added by 100 µL per each well of the plate prepared in item 5. As a result, the concentration of the test substance was diluted 2-fold, and the maximum final concentration of the substance was 200 µg/mL.
9. Culture supernatant was recovered from each well 6 hr after the start of the culture of iPS-ML2. The recovered supernatant was preserved at -80°C until use for ELISA.
10. The amount of each cytokine in the recovered culture supernatant was measured according to the protocols of IL-6 ELISA KIT (BIOLEGEND, ELISA MAX^{™} DELUXE SETHUMAN IL-6) and IL-8 ELISA KIT (BIOLEGEND, ELISA MAX^{™} DELUXE SET HUMAN IL-8) and using a plate reader (TECAN, Infinite 200PRO M nano).

**[Table 1] test substance and solution used**

| ID | test substance | solution |
|---|---|---|
| P1 | 2,4-Dinitrochlorobenzene | DMSO |
| P2 | 4-Phenylenediamine | EtOH |
| P3 | Nickel sulfate | PBS |
| P4 | 2-Mercaptobenzothiazole | EtOH |
| P5 | R(+) Limonene | EtOH |
| P6 | Imidazolidinylurea | PBS |
| N7 | Lactic acid | DMSO |

### (results)

As the result of the test, N7 did not show production of IL-6 and IL-8 6 hr after addition to iPS-ML2.

On the other hand, P1, P3, and P4 showed not less than 1.5 times higher production of IL-6 and IL-8 than the negative control containing the medium alone without a test substance, 6 hr after the addition to iPS-ML2. In addition, P2, P5, and P6 showed not less than 1.5 times higher production of IL-8 than the negative control containing the medium alone without a test substance, 6 hr after the addition to iPS-ML2. In Fig. 1, those with a production amount of 1.5 times or more compared to the negative control containing the medium alone without a test substance are marked with P (showing skin sensitizing property and/or pyrogenic property) and those with a production amount of less than 1.5 times are marked with N (not showing skin sensitizing property and/or pyrogenic property).

The results of this evaluation were compared with the results of each test substance in the conventional evaluation methods (LLNA, h-CLAT) (see Fig. 1). The evaluation results (LLNA potency) of LLNA were quoted from ATLA 38, 275-284, 2010. When skin sensitizing property is absent, Non-sensitizer is indicated, when skin sensitizing property is weak, weak is indicated, and as the strength of the skin sensitizing property increases, moderate, strong, or extreme is indicated. The results of h-CLAT (h-CLAT CD86 and CD54) were quoted from ATLA 38, 275-284, 2010, in which positive (P) is indicated for 150% or more (CD86) or 200% or more (CD54) increase in the expression with respect to the negative control, and negative (N) is indicated for lower levels. In this case, the skin sensitizing property is acknowledged when one of CD86 and CD54 is positive.

It has been confirmed that P1 to P6 have skin sensitizing property in LLNA, and h-CLAT also showed an increase in the expression level of CD86 and/or CD54 in the cells to which P1 to P6 were added. Therefore, the results correlated with the results in h-CLAT were obtained in this evaluation.

Accordingly, it is considered that the skin sensitizing property and/or pyrogenic property of each test substance can be evaluated by adding the test substance to iPS-ML2 prepared by the method described in this Example, culturing same for 6 hr, and measuring the production amount of IL-6 and/or IL-8 in the resulting culture supernatant.

Similar evaluations of the skin sensitizing property and/or the pyrogenic property were performed on different cell lots, and no difference in the results of the skin sensitizing property and/or the pyrogenic property was found between respective lots. That is, high reproducibility of this evaluation method has been confirmed.

Since this evaluation can be conducted based on the measurement by ELISA, the measurement can be performed by a method more convenient than the conventional methods (e.g., h-CLAT). Thus, for example, this evaluation is considered be also usable as a screening test before conducting the test by a conventional method.

### (Example 5) Detection test of skin sensitizer and/or pyrogen in a sample by using immortalized human monocyte

A detection test of skin sensitizer and/or pyrogen in a sample was performed according to the following procedures. In the test, iPS-ML2 was used. Cryopreserved iPS-ML2 was thawed at room temperature, washed, seeded in T-25 flask (Thermo Fisher Scientific Inc) containing 10 mL of a medium (10% FCS-containing α-MEM (GIBCO)), passaged not less than 3 times, and subjected to the test.
1. The iPS-ML2 floating in the T-25 flask was transferred to a 15 mL tube. Thereafter, centrifugation was performed at room temperature, 300×G for 5 min, and the supernatant was discarded.
2. To the pellets of iPS-ML2 was added 10 mL of PBS (Fujifilm Wako Pure Chemical Industries, Ltd.), the mixture was centrifuged at room temperature, 300×G for 5 min, and the supernatant was discarded. This operation was repeated once more.
3. The iPS-ML2 after washing was suspended in a medium (10% FCS-containing α-MEM (GIBCO)).
4. Cell number was counted using trypan blue.
5. The iPS-ML2 was seeded to the cell number of 1×10⁴/100 µL per each well of 96-well flat plate (Coring).
6. As a sample containing a skin sensitizer and/or a pyrogen, LPS and SAC were dissolved and suspended in a solution. In the case of LPS, this solution was adjusted with a medium (10% FCS-containing α-MEM (GIBCO)) to a concentration of 2000000 pg/mL. In the case of SAC, it was diluted 5-fold with a medium (10% FCS-containing α-MEM (GIBCO)).
7. 2000000 pg/mL LPS and 5-fold diluted SAC were diluted 10-fold with a medium (10% FCS-containing α-MEM (GIBCO)), and a total of 10 concentrations were prepared.
8. A sample containing a skin sensitizer and/or a pyrogen at each concentration was added by 100 µL per each well of the plate prepared in item 5. As a result, the concentration of the skin sensitizer and/or the pyrogen was diluted 2-fold, the maximum final concentration of LPS was 1000000 pg/mL, and the minimum dilution rate of SAC was 10-fold.
9. Culture supernatant was recovered from each well 6 hr and 24 hr after the start of the culture of iPS-ML2. The recovered supernatant was preserved at -80°C until use for ELISA.
10. The amount of each cytokine in the recovered culture supernatant was measured according to the protocols of IL-6 ELISA KIT (BIOLEGEND, ELISA MAX^{™} DELUXE SETHUMAN IL-6) and IL-8 ELISA KIT (BIOLEGEND, ELISA MAX^{™} DELUXE SET HUMAN IL-8) and using a plate reader (TECAN, Infinite 200PRO M nano).

### (results)

The production amounts of IL-6 and IL-8 in the culture supernatant of iPS-ML2 after adding LPS or SAC at each concentration to iPS-ML2 and culturing same for 6 hr and 24 hr are shown in Fig. 2.

For LPS, the production amounts of IL-6 and IL-8 in the culture supernatant increased in a concentration-dependent manner with respect to the negative control containing a culture medium alone, both 6 hr and 24 hr after culturing (see Fig. 2A and Fig. 2B). In Fig. 2A and Fig. 2B, the negative control is shown as a circle (negative control after 6 hr of culture) and a square (negative control after 24 hr of culture) on the vertical axis of the graph.

For SAC, the production amounts of IL-6 and IL-8 in the culture supernatant increased as the dilution rate became smaller, i.e., as the concentration became higher, with respect to the negative control containing a culture medium alone, both 6 hr and 24 hr after culturing (see Fig. 2C and Fig. 2D). Similar to the aforementioned LPS, in Fig. 2C and Fig. 2D, the negative control is shown as a circle (negative control after 6 hr of culture) and a square (negative control after 24 hr of culture) at the right end of the graph.

Accordingly, it is considered that a skin sensitizer and/or a pyrogen can be detected by adding a sample containing the skin sensitizer and/or the pyrogen to iPS-ML2 prepared by the method described in this Example, culturing same for 6 hr and/or 24 hr, and measuring the production amount of IL-6 and/or IL-8 in the resulting culture supernatant.

The detection of a skin sensitizer and/or a pyrogen by this detection method was found to show detection performance the same as or better than the detection limit of the conventional tests (e.g., PBMC test). A similar detection test was performed on different cell lots, and no difference in the results was found between respective lots. That is, high reproducibility of this detection method has been shown.

### (Example 6) Cytokine production evaluation test using functional beverage using immortalized monocyte strain

Cytokine production from immortalized monocyte strain by functional beverage was evaluated. The iPS-ML2 prepared in Example 3 was used for the test. In addition, the following functional beverages were used as test samples.

**[Table 2]**

| | functional beverages | manufacturer |
|---|---|---|
| 1 | iMUSE (registered trade mark) | Kirin Beverage Company, Limited |
| 2 | L-92 (registered trade mark) | ASAHI SOFT DRINKS CO., LTD. |
| 3 | premium gasseri bacteria | ASAHI SOFT DRINKS CO., LTD. |
| 4 | R-1 (registered trade mark) | Meiji Co., Ltd. |
| 5 | Labre (registered trade mark) Light | KAGOME CO., Ltd. |
| 6 | yogurina (registered trade mark) | SUNTORY FOODS LIMITED |
| 7 | lactobacillus helve yogurt | MEGMILK SNOW BRAND Co., Ltd. |
| 8 | Drinking Euglena (registered trade mark) | Euglena Co., Ltd. |
| 9 | Almond Effect (registered trade mark) | EZAKI GLICO CO., LTD. |
| 10 | Sugoi Daizu (registered trade mark) | Otsuka Foods Co., Ltd. |
| 11 | AMASAKE | HAKKAISAN BREWERY CO., LTD. |

The test was specifically performed according to the following procedures.
1. Frozen iPS-ML2 was thawed at room temperature, to the pellets was added 10 mL of PBS (Fujifilm Wako Pure Chemical Industries, Ltd.), the mixture was centrifuged at room temperature, 300×G for 5 min, and the supernatant was discarded. This operation was repeated once more.
2. The iPS-ML2 after washing was suspended in 10 mL of 10% FBS (Cytiva Inc., Cat#: SH30088.03) medium or a culture medium obtained by adding 2 or 5% HPL (Human platelet lysate, human platelet-derived supplement (Advantacell, Inc., Cat#: HPCHXCGL50)) to α-MEM (Gibco BRL).
3. Cell number was counted using trypan blue.
4. The iPS-ML2 was seeded to the cell number of 1×10⁴/100 µL per each well of 96-well flat plate (Corning).
5. As a sample, various commercially available functional beverages shown in Table 2 were diluted 2-fold with a medium (10% FCS-containing α-MEM (GIBCO)), and a total of 11 concentrations from the stock solution (100%) to 0.05% were prepared.
6. The various functional beverages at each concentration were added by 100 µL per each well of the plate prepared in item 5. As a result, the concentration of the various functional beverages was diluted 2-fold, the maximum final concentration was 50%, and the minimum final concentration was 0.025%.
7. iPS-ML2 was cultured at 37°C in a 5% CO₂ incubator, and the culture supernatant was recovered from each well 24 hr after the start of the culture. The recovered supernatant was preserved at -80°C until use for ELISA.
8. The amount of each cytokine in the recovered culture supernatant was measured according to the protocol of IL-6 ELISA KIT (BIOLEGEND, ELISA MAX^{™} DELUXE SETHUMAN IL-6, Cat#:430504) and using a plate reader (TECAN, Infinite 200PRO M nano).
9. The production amount of IL-6 was shown as a production amount ratio (Stimulation Index), and the production amount ratio (Stimulation Index) was calculated by the following formula. As a negative control, a medium was used.

Stimulation Index=(measured value of IL-6 production amount in cell supernatant added with various functional beverages)/(measured value of IL-6 production amount of negative control)

### (results)

The production amount ratio of IL-6 in the culture supernatant of iPS-ML2 after adding various functional beverages at each concentration to iPS-ML2 and culturing same for 24 hr is shown in Fig. 3. The horizontal axis of the graph shows sample concentration (%), and the vertical axis shows the production amount ratio with respect to the production amount (=1) of the negative control. In Fig. 3, the negative control is shown as a square on the vertical axis of the graph.

For example, when iMUSE (registered trade mark) was used as a sample, the IL-6 production amount ratio was about 17 times when it was added at a concentration of 6.25%. From this result, it was found that the maximum production amount of IL-6 is different among various samples, and that the production amount of IL-6 changes depending on the dilution concentration of the sample. It was also found that under high sample concentration conditions, for example, when the final concentration of the sample was around 50%, sufficient evaluation results could not be observed in many groups due to changes in the osmotic pressure.

Therefore, from this example, it was shown that the prepared iPS-ML2 cells can be used to measure the amount of cytokines such as IL-6 produced by immune cells, and to evaluate the degree of inflammation induction and screening by using same as an index.

### (Example 7) Comprehensive evaluation test of cytokine produced by functional beverage by using immortalized monocyte strain

Comprehensive evaluation test of cytokine produced by a sample was performed according to the following procedures. In the test, iPS-ML2 was used. Cryopreserved iPS-ML2 was thawed at room temperature, washed, seeded in T-25 flask (Thermo Fisher Scientific Inc) containing 10 mL of a medium (10% FCS-containing α-MEM (GIBCO)), passaged not less than 3 times, and subjected to the test.
1. The iPS-ML2 floating in the T-25 flask was transferred to a 15 mL tube. Thereafter, centrifugation was performed at room temperature, 300×G for 5 min, and the supernatant was discarded.
2. To the pellets of iPS-ML2 was added 10 mL of PBS (Fujifilm Wako Pure Chemical Industries, Ltd.), the mixture was centrifuged at room temperature, 300×G for 5 min, and the supernatant was discarded. This operation was repeated once more.
3. The iPS-ML2 after washing was suspended in 10 mL of a medium (10% FCS-containing α-MEM (GIBCO)) or a culture medium obtained by adding 2 or 5% HPL (Human platelet lysate, human platelet-derived supplement (Advantacell, Inc., Cat#: HPCHXCGL50)) to α-MEM (Gibco BRL).
4. Cell number was counted using trypan blue.
5. The iPS-ML2 was seeded to the cell number of 1×10⁴/100 µL per each well of 96-well flat plate (Corning).
6. A functional beverage (iMUSE (registered trade mark)) was adjusted with a medium (10% FCS-containing α-MEM (GIBCO)) to a final concentration of 10%, and added by 100 µL per one well of the plate prepared in item 5.
7. iPS-ML2 was cultured at 37°C in a 5% CO₂ incubator, and the culture supernatant was recovered from each well 6 hr and 24 hr after the start of the culture. The recovered supernatant was preserved at -80°C until use for ELISA.
8. The amount of each cytokine in the recovered culture supernatant was measured according to the protocol of Bio-Plex Pro^{™} human cytokine screening 48-Plexpanel (Bio-Rad Laboratories, Inc., Cat#: 12007283) and using a measurement device (product name: Bio-Plex 200, Bio-Rad Laboratories, Inc.).
9. The production amount of each cytokine was shown as a production amount ratio (Stimulation Index), and the production amount ratio was calculated by the following formula. As a negative control, a medium was used.

Stimulation Index=(measured value of cytokine production amount in cell supernatant added with iMUSE (registered trade mark))/(measured value of cytokine production amount of negative control)

### (results)

A comparison of the cytokine production amounts by the addition of iMUSE (registered trade mark) is shown in Table 3. In Table 3, + means that the production amount ratio is 2.5-5 times, ++ means that the production amount ratio is 5-10 times, and +++ means that the production amount ratio is not less than 10 times.

**[Table 3]**

| incubation time | | 6h | 24h |
|---|---|---|---|
| | | Stimulating effect | Stimulating effect |
| sample | | iMuse | iMuse |
| cytkine | IL-6 | + | +++ |
| | IL-8 | ++ | +++ |
| | MIP-1a | ++ | ++ |
| | MIP-1b | + | ++ |
| | IL-12(p40) | +++ | + |
| | GRO-a | - | + |
| | TNF-a | - | + |
| | MCP-1(MGAF) | + | + |
| | IFN-g | - | + |
| | CTACK | - | - |
| | IL-lb | - | - |
| | IL-10 | - | - |
| | IL-2 | - | - |
| | IL-12(p70) | - | - |
| | IL-18 | - | - |
| | IP-10 | - | - |
| | RANTES | - | - |

For example, in the case of IL-8, the production amount after 24 hr was not less than 10 times (+++). As a result, it was found that cytokines such as IL-8 and the like are also produced in addition to IL-6 shown in Example 6.

Therefore, it was shown that various cytokine productions by immune cells can be evaluated using the produced iPS-ML2 cells, and that cytokine productivity of various substances can be evaluated using the cytokine evaluation.

## Claims

1. A method for evaluating the skin sensitizing property and/or the pyrogenic property of a test substance, comprising measuring an amount of IL-6 and/or IL-8 produced in a culture medium by culturing human immortalized myeloid cells in the presence of the test substance.

2. The method according to claim 1, wherein the human immortalized myeloid cell is a human immortalized monocyte or a dendritic cell prepared from the human immortalized monocyte.

3. The method according to claim 1 or 2, wherein a concentration of the test substance during the culture of the human immortalized myeloid cells includes at least 3 kinds of concentration between higher than 0 µg/mL and not more than 1000 µg/mL.

4. The method according to any one of claims 1 to 3, wherein a time of the culture is at least one kind selected from 3 hr to 48 hr.

5. The method according to any one of claims 1 to 4, wherein the production amount is measured by an immunological measurement method.

6. The method according to claim 5, wherein the immunological measurement method is ELISA.

7. The method according to any one of claims 1 to 6, further comprising determining that the test substance has skin sensitizing property and/or pyrogenic property when the production amount is not less than 1.5 times the production amount of a negative control.

8. A method for detecting a skin sensitizer and/or a pyrogen in a sample, comprising measuring an amount of IL-6 and/or IL-8 produced in a culture medium by culturing human immortalized myeloid cells in the presence of the sample.

9. The method according to claim 8, wherein the human immortalized myeloid cell is a human immortalized monocyte or a dendritic cell prepared from the human immortalized monocyte.

10. The method according to claim 8 or 9, wherein a concentration of the sample during the culture of the human immortalized myeloid cells includes at least 3 kinds of concentrations.

11. The method according to any one of claims 8 to 10, wherein time of the culture is at least one kind selected from 3 hr to 48 hr.

12. The method according to any one of claims 8 to 11, wherein the production amount is measured by an immunological measurement method.

13. The method according to claim 12, wherein the immunological measurement method is ELISA.

14. The method according to any one of claims 8 to 13, further comprising determining that the sample comprises a skin sensitizer and/or a pyrogen when the production amount increased than the production amount of a negative control.

15. The method according to any one of claims 8 to 14, wherein the skin sensitizer and/or pyrogen are/is lipopolysaccharide (LPS) and/or Staphylococcus aureus Cowan 1 (SAC).

16. A method for evaluating an action of a sample on a function of immune cells, comprising measuring an amount of cytokine produced in a culture medium by culturing human immortalized myeloid cells in the presence of the sample.

17. The method according to claim 16, wherein the human immortalized myeloid cell is a human immortalized monocyte or a dendritic cell prepared from the human immortalized monocyte.

18. The method according to claim 16 or 17, wherein the cytokine is selected from the group consisting of IL-1α, IL-1b, IL-1ra, IL-2, IL-2Ra, IL-3, IL-4, IL-5, IL-6, IL-7, IL-8, IL-9, IL-10, IL-12(p70), IL-12(p40), IL-13, IL-15, IL-16, IL-17A, IL-18, CTACK, Eotaxin, FGF basic, G-CSF, GM-CSF, GRO-α, HGF, IFN-α2, IFN-γ, IP-10, LIF, MCP-1(MCAF), MCP-3, M-CSF, MIF, MIG, MIP-1α, MIP-1β, β-NGF, PDGF-BB, RANTES, SCF, SCGF-β, SDF-1a, TNF-α, TNF-β, TRAIL, and VEGF-A.

19. The method according to any one of claims 16 to 18, wherein the action on the function of immune cells is selected from an action to increase cytokine production from immune cells, an action to decrease cytokine production from immune cells, an inflammation-inducing action by immune cells, an inflammation-suppressing-inducing action by immune cells, an immunity activation-inducing action by immune cells, an immunity suppression-inducing action by immune cells, a mastocyte-stimulating action by immune cells, an IgE production-enhancing action by immune cells, an IgE production-suppressing action by immune cells, an IgA production-enhancing action by immune cells, a lymphocyte-proliferating and activating action by immune cells, an eosinophil production-increasing action by immune cells, a leukocyte migration-promoting action by immune cells, an apoptosis-inducing action by immune cells, an apoptosis-suppressing action by immune cells, a virus resistant-increasing action by immune cells, a cell proliferation-promoting action by immune cells, an angiogenesis inducing action by immune cells, a wound-healing action by immune cells, a hematopoietic cell proliferation-promoting action by immune cells, a progenitor cell differentiating action by immune cells, and a cell differentiation-inhibiting action by immune cells.

20. The method according to any one of claims 16 to 19, wherein a time of the culture is 3 hr to 48 hr.

21. The method according to any one of claims 16 to 20, further comprising determining that the sample has functionality when the production amount of the cytokine in the culture medium obtained by culturing the human immortalized myeloid cells in the presence of the sample is not less than 2 times the production amount of cytokine in a culture medium obtained by culturing the human immortalized myeloid cells in the presence of a negative control.

22. The method according to any one of claims 16 to 21, wherein the sample is a functional beverage.

23. A kit for use in the method according to any one of claims 1 to 22, comprising a human immortalized monocyte or a dendritic cell prepared from the human immortalized monocyte.
